# EUROPEAN PATENT APPLICATION

(11) **EP 2 314 197 A1**
(43) Date of publication of application: **27.04.2011**
(21) Application number: 08791912.2
(22) Date of filing: 30.07.2008
(51) Int. Cl.: A61B 1/00

(54) **OPTICAL ELEMENT, SPECTROSCOPIC ELEMENT, OPTICAL UNIT AND OPTICAL DEVICE**

(71) Applicant: Olympus Corporation, Shibuya-ku Tokyo 151-0072 (JP)
(72) Inventor: UZAWA, Kunihiko, Tokyo 151-0072 (JP)
(74) Representative: Schicker, Silvia
(86) International application number: PCT/JP2008/063681
(87) International publication number: WO 2010/013326

(57) **Abstract**

An optical element includes a multilayer film which has a first transmittance characteristic in a first wavelength region, and has a second transmittance characteristic in a second wavelength region existing at the side where a wavelength is longer than that of the first wavelength region. The multilayer film has a transmittance characteristic where a first peak with a maximum transmittance exists in the second wavelength region.

## Description

### Technical Field

The present invention relates to an optical element, a spectroscopic element, an optical unit, and an optical device including the optical unit.

### Background Art

Endoscopic devices for performing diagnoses of lesions occurring in a living body are widely well known. One of such endoscopic devices includes, for example, a fluorescence endoscope device. This fluorescence endoscope device diagnoses the lesion areas occurring in the biological tissues on the basis of information included in the acquired fluorescence images. The fluorescence endoscope device irradiates the biological tissue surface which is a body to be irradiated with excitation light, and excites fluorescent substances included in the biological tissue. The fluorescence image is acquired by capturing the fluorescence emanated from the biological tissue.

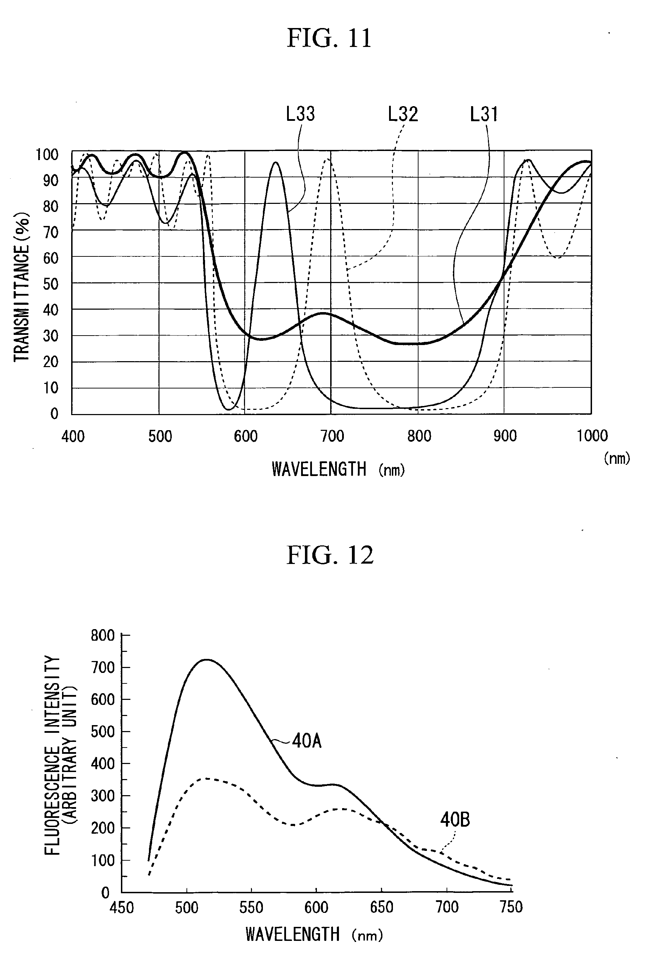

When autofluorescence from the biological tissue surface is detected by irradiating the biological tissue surface with excitation light, it is known that normal biological tissue (hereinafter, referred to as normal tissue) and biological tissue including the lesion area (hereinafter, referred to as lesion tissue) are different from each other in intensity of autofluorescence. FIG. 12 is a graph showing the relationship between the wavelength of excitation light and the intensity of autofluorescence emanated from the biological tissue. The wavelength-dependent intensity distribution of the autofluorescence emanated from the normal tissue when the normal tissue is irradiated with excitation light is shown by the solid line 40A, and the wavelength-dependent intensity distribution of the autofluorescence emanated when the lesion tissue is irradiated with excitation light is shown by the dashed line 40B (see Nonpatent Document 1 below). As shown in FIG. 12, the fluorescence intensity distribution of the lesion tissue shown by the dashed line 40B is obviously different from the fluorescence intensity distribution of the normal tissue shown by the solid line 40A. That is, the fluorescence intensity distribution is acquired from the autofluorescence image of the biological tissue, and such fluorescence intensity distribution is analyzed, so that it is possible to determine whether such a biological tissue includes the lesion area. -

For example, the biological tissue is formed to have a layer structure, and includes a submucosa, and a mucosa membrane located above this submucosa. The submucosa includes a lot of collagen or elastin which emanates the autofluorescence on irradiation of excitation light. When a lesion occurs in the biological tissue, the layer structure changes. Therefore, until the autofluorescence emanated from collagen or elastin reaches the epithelium, the autofluorescence is strongly influenced by changes in the layer structure and thus it is attenuated. Consequently, by detecting the fluorescence intensity of the wavelength region including a main wavelength (for example, 500 nm to 550 nm) of the autofluorescence emanated from collagen or elastin, it is possible to acquire information for discerning whether the biological tissue includes a lesion area, and where in the biological tissue the lesion area exists.

In addition, it is known that porphyrin which is an organic compound existing in the living body tends to be accumulated in a tumor. When the biological tissue including a lot of porphyrin is irradiated with excitation light of the wavelength region of blue to green similarly to collagen or elastin, a peak exists in a region with a wavelength of around 630 nm, in the intensity distribution of the autofluorescence emanated from such a biological tissue. Consequently, by detecting the fluorescence intensity of the wavelength region including a main wavelength (that is, around 630 nm) of the autofluorescence emanated from porphyrin, it is possible to acquire information for discerning whether a tumor is generated in the biological tissue, and where in the biological tissue a tumor is generated.
An optical unit having a predetermined spectral transmittance characteristic is used in detection of the above-mentioned fluorescence intensity. The predetermined spectral transmittance characteristic means a spectral transmittance characteristic of transmitting fluorescence of the narrow wavelength region including, for example, 630 nm. As seen from the above, a variety of information related to the biological tissue is included in the autofluorescence spectrum from the biological tissue for each wavelength region.

In FIG. 12, the fluorescence intensity distribution of the normal tissue shown by the solid line 40A, and the fluorescence intensity distribution of the lesion tissue shown by the dashed line 40B have little difference therebetween in the wavelength region around 630 nm, but have a significant difference therebetween in the wavelength region of 500 nm to 550 nm. Such a difference of the intensity for each wavelength region is speculated to be caused by the progress conditions of the lesion or the lesion generation area and the like. Therefore, it is considered that acquisition of the fluorescence intensities for each wavelength region having different autofluorescence spectrums and comparison therebetween are useful for a diagnosis of the lesion.

As seen from the above, when performing a spectroscopic analysis of the autofluorescence spectrum of the biological tissue, and extracting information included in each of the spectral bands, it is possible to perform a diagnosis of the lesion. In the past, the spectroscopic analysis of the autofluorescence spectrum has been performed using an etalon-type spectroscopic element as the optical element (for example, see Patent Document 1 below).
As disclosed in Patent Document 1, a conventional etalon-type spectroscopic element has a peak with a maximum transmittance in the wavelength region which is spectroscopically scanned, and has a transmittance characteristic where the transmittance decreases before and after the peak.
[Patent Document 1] Japanese Patent Application, First Publication No. 2006-25802
[Nonpatent Document 1] Life Science Opened by Series Light, Vol. 6, Medicine Diagnosis through Light, Editor in Charge: Tamura Mamoru, Kyoritsu Shuppan, March 30, 2001, page 91

### Disclosure of Invention

### Technical Problem

In recent years, it has been preferable to increase the amount of transmitted light obtained by the etalon-type spectroscopic element, from the view point of improving diagnostic accuracy for the lesions and the like. However, since the conventional etalon-type spectroscopic element has a small full-width at half-maximum (wavelength width where the transmittance is 50% of a peak value) in the peak, it is difficult to increase the amount of transmitted light.
The invention is devised in view of such circumstances, and an object of the present invention is to provide an optical element which has a large full-width at half-maximum in a peak, and is capable of being suitably used in a spectroscopic analysis. Further, another object of the present invention is to provide a spectroscopic element in which such an optical element is used, an optical unit, and an optical device in which the optical unit is used.

### Technical Solution

An optical element of the invention includes a multilayer film which has a first transmittance characteristic in a first wavelength region, and has a second transmittance characteristic in a second wavelength region existing at the side where a wavelength is longer than that of the first wavelength region. The multilayer film has a transmittance characteristic where a first peak with a maximum transmittance exists in the second wavelength region.
The optical element of the invention has a peak of the transmittance in the second wavelength region equivalent to the second transmittance characteristic, when the transmittance is measured. When such optical elements are disposed opposite to each other, the full-width at half-maximum in the peak of the transmittance generated in the second wavelength region increases.

In the optical element of the invention, the transmittance in the first peak may be higher than the lowest transmittance in the second wavelength region, and the difference thereof may be 2% or more.
In this optical element, when the transmittance is measured, the transmittance of the peak in the second wavelength region equivalent to the second transmittance characteristic is 2% or more higher than that of a region having the lowest transmittance before and after the peak. When such optical elements are disposed opposite to each other, the full-width at half-maximum in the peak of the transmittance generated in the second wavelength region increases.

Moreover, in the optical element of the invention, when the two optical elements are disposed opposite to each other, these two optical elements have a third transmittance characteristic in a third wavelength region, and have a fourth transmittance characteristic in a fourth wavelength region existing at the side where the wavelength is longer than that of the third wavelength region, and the first peak of the optical element may exist in the fourth wavelength region.
In this optical element, the transmittance of the third transmittance characteristic in the third wavelength region obtained in the state where a pair of optical elements are disposed opposite to each other is high, and the full-width at half-maximum in the peak of the transmittance generated in the fourth wavelength region increases.

A spectroscopic element of the invention includes two optical elements. The two optical elements are disposed opposite to each other, and the spectroscopic element has a third transmittance characteristic in a third wavelength region, and has a fourth transmittance characteristic in a fourth wavelength region existing at the side where a wavelength is longer than that of the third wavelength region. The first peak of the optical element exists in the fourth wavelength region of the spectroscopic element.
In this spectroscopic element, the transmittance of the third transmittance characteristic in the third wavelength region is high, and the full-width at half-maximum in the peak of the transmittance generated in the fourth wavelength region increases.

An optical unit of the invention includes the two optical elements disposed opposite to each other, and an adjustment mechanism that adjusts a gap along the light path of the optical elements. The adjustment mechanism performs switching of a wavelength in a wavelength region which has a higher transmittance than the lowest transmittance in the second wavelength region and includes the first peak.

The optical unit of the invention may have the third transmittance characteristic in the third wavelength region, and may have a fourth transmittance characteristic where a second peak with a maximum transmittance exists at the fourth wavelength region of the longer wavelength side than that of the third wavelength region. In this optical unit, the full-width at half-maximum in the second peak is 30 nm or more, and the lowest transmittance in the fourth wavelength region is 1% or less.

The optical unit of the invention is configured such that the full-width at half-maximum in the peak of the transmittance generated in the fourth wavelength region is 30 nm or more, and can make the amount of transmitted light increase. On the other hand, since the lowest transmittance is 1% or less before and after the peak of the transmittance generated in the fourth wavelength region, it is possible to reduce the amount of transmission of the light that causes noise.

The optical device of the invention includes the optical unit, a light source unit that irradiates an object with illumination light, an optical system that forms an image of the object, and an imaging unit that captures the image, wherein the optical unit is disposed between the optical system and the imaging unit.

In the optical device of the invention, the optical unit may be an etalon-type spectroscopic element. In this case, an optical element of the optical unit may transmit fluorescence emanated from an object, and the light source unit may irradiate the object with excitation light for exciting the fluorescence.
The optical device of the invention irradiates the object with illumination light from the light source unit, acquires fluorescence through autofluorescence to the imaging unit, and performs a diagnosis of the lesion and the like. The above-mentioned optical unit is included in the imaging unit, so that it is possible to increase the amount of transmitted light in the fourth wavelength region by the fourth transmittance characteristic.

### Advantageous Effects

According to the optical element of the invention, the peak is generated in the wavelength region showing the second transmittance characteristic. For this reason, when the optical elements are disposed opposite to each other, it is possible to increase the full-width at half-maximum in the peak of the fourth transmittance characteristic in the optical unit of the invention. As a result, it is possible to increase the amount of transmitted light in this wavelength region.
In addition, according to the optical device of the invention, it is possible to increase the amount of autofluorescence transmitted light from the autofluorescence of the biological tissue, and thus the diagnostic accuracy based on the autofluorescence can be improved.

### Brief Description of Drawings

FIG. 1 is a diagram illustrating the overall configuration of a fluorescence endoscope device including an optical unit according to an embodiment of the invention.
FIG. 2 is a schematic configuration diagram illustrating the optical unit according to the embodiment of the invention.
FIG. 3 is a graph illustrating the transmittance characteristic of an optical filter according to the embodiment of the invention.
FIG. 4 is a diagram illustrating a configuration of an etalon-type spectroscopic element which is the optical unit.
FIG. 5 is a graph illustrating the transmittance characteristic of a single optical element.
FIG. 6 is a schematic diagram showing the transmittance characteristic of the etalon-type spectroscopic element.
FIG. 7 is a diagram illustrating a structure of an optical element according to example 1, and a structure of a conventional optical element as a comparative example.
FIG. 8 is a diagram illustrating the transmittance characteristic of the optical element according to example 1, and the transmittance characteristic on the combination of a pair of optical elements.
FIG. 9 is a diagram illustrating the transmittance characteristic of the conventional optical element in the comparative example, and the transmittance characteristic on the combination of a pair of optical elements.
FIG. 10 is a diagram illustrating a structure of the optical element according to example 2.
FIG. 11 is a diagram illustrating the transmittance characteristic of the optical element according to example 2, and the transmittance characteristic of a combination of a pair of optical elements.
FIG. 12 is a graph illustrating an example of autofluorescence spectra of a normal tissue and a lesion tissue.

### Explanation of Reference

1: OPTICAL UNIT
2: FLUORESCENCE ENDOSCOPE DEVICE (OPTICAL DEVICE)
3: IMAGING SECTION
5: LIGHT SOURCE SECTION (LIGHT SOURCE UNIT)
13: OBJECTIVE OPTICAL SYSTEM (OPTICAL SYSTEM)
15: LIGHT RECEIVING ELEMENT (IMAGING UNIT)
16: ETALON-TYPE SPECTROSCOPIC ELEMENT (A PAIR OF OPTICAL ELEMENTS)
17: VARIABLE MECHANISM (ADJUSTMENT MECHANISM)
31,32: OPTICAL ELEMENT
34: MULTILAYER FILM
LG: AIR LAYER
WL1: FIRST WAVELENGTH REGION
WL2: SECOND WAVELENGTH REGION
WL3: THIRD WAVELENGTH REGION
WL4: FOURTH WAVELENGTH REGION

### Best Mode for Carrying out the Invention

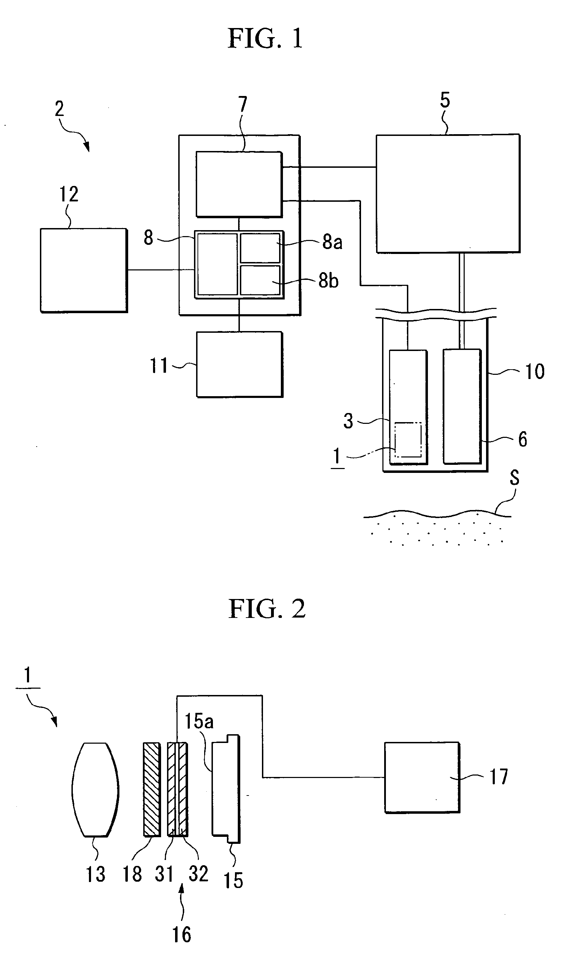

An embodiment of the invention will be described with reference to the drawings.
As shown in FIG. 1, an optical unit 1 is disposed within an imaging section 3 of a fluorescence endoscope device (optical device) 2. This fluorescence endoscope device 2 includes the imaging section 3 with the built-in optical unit 1; a light source section (light source unit) 5; an illumination section (light source unit) 6; a control section 7 for controlling the imaging section 3, the light source section 5 and the illumination section 6; and an image processing section 8.

The imaging section 3 and the illumination section 6 are disposed at the leading end of an insertion section 10 of the fluorescence endoscope device 2. The illumination section 6 is connected to the light source section 5 through an optical transmission means such as a light guide which is not shown in the drawing. Light emanated from the light source section 5 is applied to the biological tissue surface S through the illumination section 6.

The imaging section 3 and the light source section 5 are connected to the control section 7. The control section 7 controls the timing of supplying excitation light generated by the light source section 5 to the illumination section 6. In addition, the control section 7 controls the timing at which the imaging section 3 captures a fluorescent image of the biological tissue surface S. An image signal to be output from the imaging section 3 is processed into the fluorescence image by the image processing section 8.

The image processing section 8 includes a memory circuit 8a and an operational circuit 8b. The memory circuit 8a temporarily stores the image signal as data. The operational circuit 8b performs operations required for image processing on the basis of the data stored in the memory circuit 8a. In addition, an external recording device 11 such as a DVD and an HDD is connected to the image processing section 8. This external recording device 11 can permanently save various types of image data. The image data include image signal data acquired by the imaging section 3, or image data processed by the image processing section 8. The image signal data recorded in the external recording device 11 are appropriately read out by the image processing section 8 and data processing is performed. The image data processed by the image processing section 8 are displayed on a display screen of a TV monitor 12 as a fluorescence image.

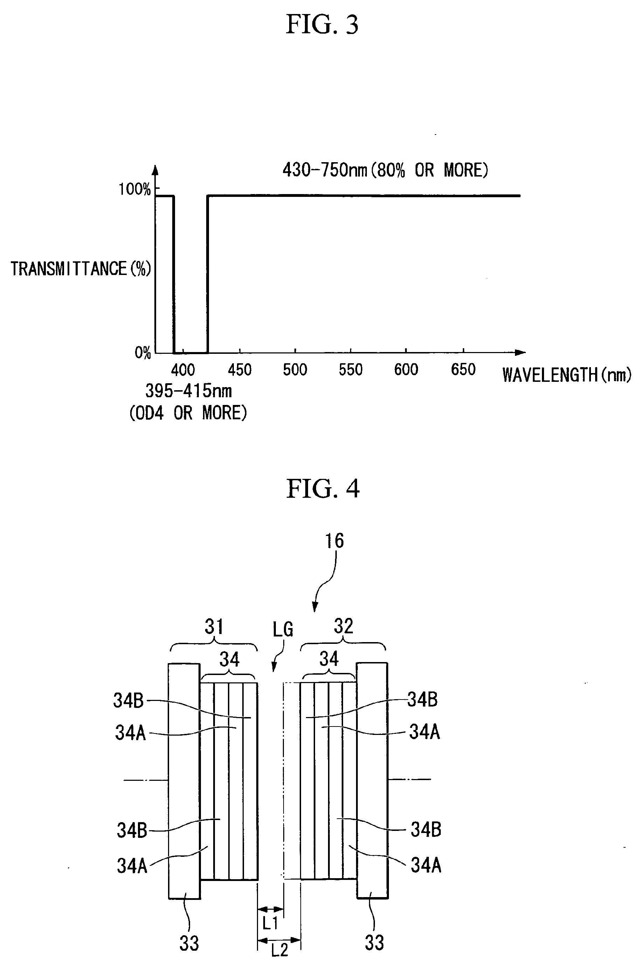

The optical unit 1 has a function of separating and detecting fluorescence emanated from the biological tissue. As shown in FIG. 2, the optical unit 1 includes an etalon-type spectroscopic element (a pair of optical elements) 16 and a variable mechanism (adjustment mechanism) 17.
The etalon-type spectroscopic element 16 is disposed between an objective optical system (optical system) 13 and a light receiving surface 15a of a light receiving element (imaging unit) 15. As shown in FIG. 4, the variable mechanism 17 changes the gap between an optical element 31 and an optical element 32 in the etalon-type spectroscopic element 16, that is, the thickness of an air layer LG between the optical element 31 and the optical element 32.

An optical filter 18 is an excitation light cutoff filter, and is disposed between the objective optical system 13 and the etalon-type spectroscopic element 16. In the embodiment, as the optical filter 18, as shown in FIG. 3, an optical filter is used which has a characteristic where OD value is 4 or more (1×10⁻⁴ or less for the transmittance) in the range of the wavelength of 395 nm to 415 nm, and the transmittance is 80% or more in the range of the wavelength of 430 nm to 750 nm.

The etalon-type spectroscopic element 16 is a Fabry-Perot etalon-type bandpass filter. A multiple beam interference action is used in spectroscopy. This etalon-type spectroscopic element 16 is capable of scanning a transmission wavelength region by adjusting the thickness of the air layer LG. Here, the term "capable of scanning" means to be capable of moving a predetermined transmission range in the transmission wavelength region. As a result, it is possible to freely transmit only light having a desired wavelength range.

As shown in FIG. 4, the etalon-type spectroscopic element 16 includes the first optical element 31 disposed perpendicular to the optical axis, and the second optical element 32 similarly disposed perpendicular to the optical axis. The second optical element 32 is disposed parallel to the first optical element 31, and the air layer LG is provided between the first optical element 31 and the second optical element 32. Each of the optical elements 31 and 32 has a transparent parallel-plate substrate 33 in the autofluorescence wavelength region, and a multilayer film 34 laminated on one side of the substrate 33. The optical elements 31 and 32 are disposed opposite each of the multilayer films 34. The multilayer film 34 has, for example, a multilayer structure with five to nine layers, and is composed of a high refractive index layer 34A and a low refractive index layer 34B which are alternately laminated. One of the high refractive index layers 34A comes into contact with one side of the substrate 33. The thicknesses of the high refractive index layer 34A and the low refractive index layer 34B are each several nm to several hundreds of nm or so. The high refractive index layer 34A and the low refractive index layer 34B are manufactured using a known sputtering method or a vapor deposition method.

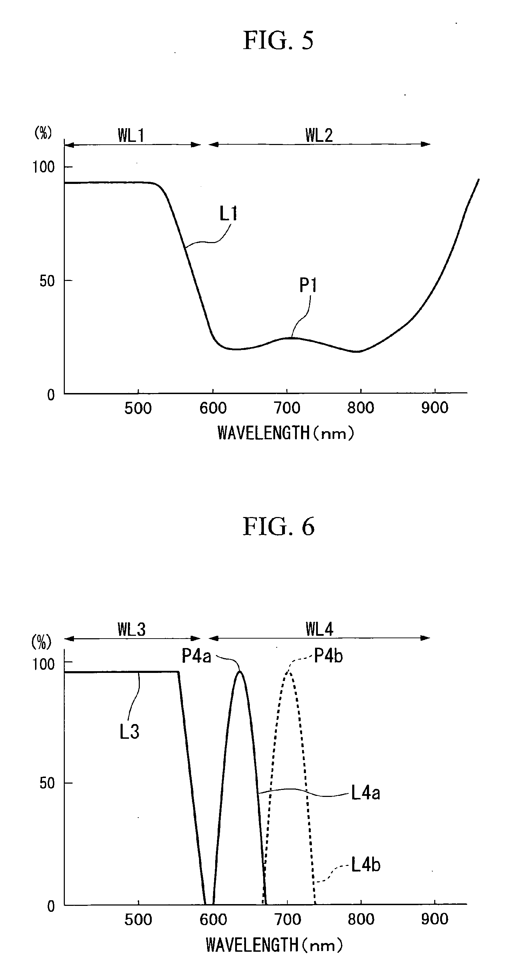

FIG. 5 shows a spectral transmittance characteristic of the single first optical element 31, or the single second optical element 32. In addition, FIG. 6 shows a spectral transmittance characteristic of the etalon-type spectroscopic element 16. The horizontal axis of each graph is a wavelength (nm), and the vertical axis thereof is a transmittance (%).
In the transmittance characteristic of FIG. 5, the entire wavelength region is divided into the first wavelength region WL1 indicating the range of the wavelength of 400 nm to 590 nm, and the second wavelength region WL2 indicating the range of the wavelength of 590 nm to 900 nm. The transmittance characteristic of the first optical element 31 (the same is true of the second optical element 32) is shown by the solid line L1 in FIG. 5. As shown in FIG. 5, the first optical element 31 has a transmittance characteristic where transmittance is 50% or more in the first wavelength region WL1, and the transmittance is 50% or less in the second wavelength region WL2. Further, the first optical element 31 has a transmittance characteristic where peak (first peak) P1 having a low transmittance exists in a region with a wavelength of around 700 nm included in the second wavelength region WL2. According to the etalon-type spectroscopic element 16 disposed opposite the optical elements 31 and 32 having such a transmittance characteristic, it is possible to increase the full-width at half-maximum (wavelength width set to 50% when the transmittance reaches the peak) in the peak. Meanwhile, the first peak is a point showing that the transmittance has a maximum value, and a point showing that the transmittance decreases around the maximum value.

In a spectral transmittance characteristic of FIG. 6, the entire wavelength region is divided into the third wavelength region WL3 indicating the range of the wavelength of about 400 nm to about 590 nm, and the fourth wavelength region WL4 indicating the range of the wavelength of about 590 nm to about 900 nm. The transmittance characteristic of the etalon-type spectroscopic element 16 in the third wavelength region WL3, that is, the third transmittance characteristic is shown by the solid line L3 in FIG. 6. The transmittance characteristic of the etalon-type spectroscopic element 16 in the fourth wavelength region WL4, that is, the fourth transmittance characteristic is shown by the solid line L4a or the dashed line L4b in FIG. 6.

First, reference is made to FIG. 6 to describe the spectral transmittance characteristic of the etalon-type spectroscopic element 16 (hereinafter, often referred to simply as the transmittance characteristic).
The fourth transmittance characteristic shown by the solid line L4a is a transmittance characteristic of the etalon-type spectroscopic element 16 when the gap (that is, the thickness of the air layer LG) between the optical element 31 and the optical element 32 is L1 (see FIG. 4). In addition, the fourth transmittance characteristic shown by the dashed line L4b is a transmittance characteristic of the etalon-type spectroscopic element 16 when the gap between the optical element 31 and the optical element 32 is L2 longer than L1. As seen from the above, when the gap between the optical element 31 and the optical element 32 changes from L1 to L2, the transmittance characteristic of the etalon-type spectroscopic element 16 in the fourth wavelength region WL4 changes from the solid line L4a to the dashed line L4b.

At the time of scanning of the transmission wavelength region, at least any one of the two optical elements 31 and 32 is moved from an initial position. For example, the first optical element 31 is fixed, and then the second optical element 32 is moved in a direction away from the first optical element 31. When the gap between the optical element 31 and the optical element 32 is L1, the transmittance characteristic of the etalon-type spectroscopic element 16 in the third wavelength region WL3 is shown by the solid line L3. As shown in FIG. 6, the etalon-type spectroscopic element 16 has a transmittance characteristic of a transmittance of 50% or more in a region with a wavelength of about 400 nm to about 570 nm included in the third wavelength region WL3.

In addition, when the gap between the optical element 31 and the optical element 32 is L1, the transmittance characteristic of the etalon-type spectroscopic element 16 in the fourth wavelength region WL4 is shown by the solid line L4a. As shown in FIG. 6, the etalon-type spectroscopic element 16 has a transmittance characteristic where peak (second peak) P4a having a transmittance close to 100% exists in a region with a wavelength of around 630 nm included in the fourth wavelength region WL4.

In addition, the transmittance decreases around the peak P4a, and is set to zero when the wavelength is about 600 nm and about 670 nm, respectively. Therefore, the practical transmission range for the transmittance characteristic of the etalon-type spectroscopic element 16 in the fourth wavelength region WL4 is from about 600 nm to about 670 nm. In addition, the range with a transmittance set to nearly zero is from the long wavelength end (about 590 nm) of the solid line L3 to the short wavelength end (about 600 nm) of the solid line L4a, and is the long wavelength side (about 670 nm to 900 nm) of the solid line L4a. As seen from the above, the region (light blocking region) with a transmittance set to nearly zero exists around the peak P4a.

Next, the first optical element 31 is fixed, and then the second optical element 32 is moved in a direction further away from the first optical element 31. When the gap between the optical element 31 and the optical element 32 is L2, the transmittance characteristic of the etalon-type spectroscopic element 16 in the fourth wavelength region WL4 is shown by the dashed line L4b. As shown in FIG. 6, when the gap between the optical element 31 and the optical element 32 changes from L1 to L2, the peak of the transmittance of the etalon-type spectroscopic element 16 is moved to the long wavelength side. That is, the peak (second peak) of the transmittance in the fourth wavelength region WL4 shifts from P4a to P4b. On the other hand, the transmittance characteristic (solid line L3) of the etalon-type spectroscopic element 16 in the third wavelength region WL3 does not almost change. In addition, the practical transmission range for the transmittance characteristic of the etalon-type spectroscopic element 16 in the fourth wavelength region WL4 is from about 670 nm to about 740 nm. However, the width of the region thereof, that is, the difference between the maximum wavelength and the minimum wavelength of the region thereof is about 70 nm, without changing the transmittance characteristic of the etalon-type spectroscopic element 16 in the fourth wavelength region WL4 when the gap between the optical element 31 and the optical element 32 is L2.

As seen from the above, it is possible to shift the peak of the transmittance in the fourth wavelength region WL4 from P4a to P4b by changing the gap between the optical element 31 and the optical element 32 from L1 to L2, that is, changing the thickness of the air layer LG between the first optical element 31 and the second optical element 32. As a result, it is possible to perform spectroscopic scanning within a predetermined wavelength region.
In addition, even when the thickness of the air layer LG is changed at the time of scanning in this etalon-type spectroscopic element 16, it is possible to realize the transmittance characteristic where transmittance is maintained at 50% or more in the wavelength region of about 400 nm to about 570 nm, and that the size of the wavelength when the transmittance reaches the peak changes with the change in the thickness of the air layer LG in the wavelength region of about 570 nm to about 900 nm.

Meanwhile, as mentioned above, the transmittance characteristic shown in FIG. 6 can be realized by using the first optical element 31 and the second optical element 32 in pairs. The first optical element 31 and the second optical element 32 will now be described. Meanwhile, although the first optical element 31 is described by way of example, the second optical element 32 also has the same characteristic as that of the first optical element 31.
The peak P1 of the transmittance is included in the fourth wavelength region WL4 in FIG. 6. This fourth wavelength region WL4 is a region in which the spectroscopic scanning is performed. Therefore, the multilayer film of the first optical element 31 is preferably configured so that the peak P1 of the transmittance exists in the wavelength region in which the spectroscopic scanning is performed, when the first optical element 31 is used as a spectroscopic element. Thereby, it is possible to increase the full-width at half-maximum in the peak of the transmittance in the spectroscopic element in which the first optical element 31 and the second optical element 32 are used in pairs.

Moreover, in FIG. 5, the transmittance in the peak P1 is preferably 2% or more higher than the lowest transmittance in the second wavelength region WL2. Thereby, in the spectroscopic element in which the first optical element 31 and the second optical element 32 are used in pairs, it is possible to further decrease the transmittance in the region (light blocking region) having a transmittance set to nearly zero which exists around the peak P4a and around the peak P4b.

As seen from the above, in the spectroscopic element in which the first optical element 31 and the second optical element 32 are used in pairs, for example, the etalon-type spectroscopic element 16, the full-width at half-maximum in the peak P4b existing in the fourth wavelength region WL4 can be increased to, for example, 30 nm or more. In addition, the lowest transmittance in the fourth wavelength region WL4 can be set to 1 % or less.
Meanwhile, this etalon-type spectroscopic element 16 has the third transmittance characteristic where transmittance is maintained at 50% or more even when the thickness of the air layer LG is changed in the wavelength region of about 400 nm to about 570 nm, and the fourth transmittance characteristic where peak of the transmittance shifts (the wavelength at the time of the peak changes) when the thickness of the air layer LG is changed in the wavelength region of about 590 nm to about 900 nm.

Next, a procedure of acquiring an autofluorescence image of the biological tissue will be described by using the fluorescence endoscope device 2 having the optical unit 1 according to the embodiment.

First, excitation light emitted from the light source section 5 is applied to the biological tissue surface S over a given period of time. A semiconductor laser having a wavelength of 405±5 nm at the time of the peak is used in the light source section 5. The excitation light emitted from the light source section 5 includes a wavelength for exciting an autofluorescent substance. Thereby, fluorescence having mainly a wavelength of 500 nm to 550 nm is generated from the biological tissue including collagen or elastin, and fluorescence having a wavelength of around 630 nm at the time of the peak is emanated from the biological tissue including porphyrin. The fluorescence emanated from the biological tissue including collagen or elastin is included in the wavelength region WL3, and the fluorescence emanated from the biological tissue including porphyrin is included in the wavelength region WL4.

The variable mechanism 17 is caused to operate while the imaging section 3 receives the fluorescence emanated from the biological tissue, the second optical element 32 is moved from the initial position, and then the gap between the first optical element 31 and the second optical element 32 of the etalon-type spectroscopic element 16, that is, the thickness of the air layer LG is changed. Control of operation is performed using a control signal transmitted from the control section 7 of the fluorescence endoscope device 2. When the second optical element 32 is moved with respect to the first optical element 31, the transmittance characteristic of the etalon-type spectroscopic element 16 changes with this movement. When the second optical element 32 reaches the first position at which the thickness of the air layer LG is set to 520 nm, the peak P4a of the transmittance appears in a region with a wavelength of around 630 nm, as shown by the solid line L4a in FIG. 6. Subsequently, when the second optical element 32 reaches the second position at which the thickness of the air layer LG is set to 720 nm by causing the variable mechanism 17 to operate and further moving the second optical element 32, the peak P4b of the transmittance appears in a region with a wavelength of around 700 nm, as shown by the dashed line L4b in FIG. 6.

When the second optical element 32 exists in the first position (state A1), as shown in FIG. 6, the imaging plane of the imaging section 3 receives fluorescence shown by the solid line L4a in the wavelength region WL4, and fluorescence shown by the solid line L3 in the wavelength region WL3. In addition, when the second optical element 32 is set to the second position (state A2), the imaging plane of the imaging section 3 receives fluorescence shown by the dashed line L4b in the wavelength region WL4, and fluorescence shown by the solid line L3 in the wavelength region WL3. In the state A1, the fluorescence received in the imaging section 3 is accumulated in the memory circuit 8a as an image signal D1 while the state A1 is maintained. In the state A2, the fluorescence received in the imaging section 3 is accumulated in the memory circuit 8a as an image signal D2 while the state A2 is maintained.

In the fluorescence endoscope device 2, by setting a series of processes performed in the above two states A and B to one cycle, the biological tissue surface S is repeatedly irradiated with the excitation light, and two types of image signals are acquired in one cycle.

The obtained image signals (D1, D2) is processed by the operational circuit 8b of the image processing section 8. As shown in FIG. 12, since the spectrum of the autofluorescence emanated from the living body has an extremely low fluorescence intensity in a region (region reaching the transmittance of the peak P4b) with a frequency of around 700 nm, the fluorescence intensity (image signal D2) acquired in the state A2 only information on the wavelength region WL3 is substantially reflected. On the other hand, since the spectral component which is dependent on porphyrin in the autofluorescence emanated from the living body reaches the maximum value in a region (region reaching the transmittance of the peak P4a) with a frequency of around 630 nm, the fluorescence intensity (image signal D1) acquired in the state A1 is added with information in the vicinity of the peak P4a appearing in the wavelength region WL4 to the information on the wavelength region WL3. Therefore, it is possible to generate a new image signal E1 having only a fluorescent component in the vicinity of the peak P4a by performing an operation of subtracting the image signal D2 from the image signal D 1.

According to this optical unit 1, even when the second optical element 32 exists in both the first position and the second position, the transmittance characteristics of the etalon-type spectroscopic element 16 are virtually identical with each other in the wavelength region WL3. Therefore, it is possible to remove the information on the wavelength region WL3 from data including the information on both the wavelength region WL3 and the wavelength region WL4 by performing the above-mentioned operation. As a result, it is possible to extract data related only to fluorescence in the wavelength region WL4. This means that measurement only for light (fluorescence) of a desired wavelength region can be performed.
In addition, when the second optical element 32 exists in the second position, only the information on the wavelength region WL3 can be acquired, thereby allowing the information on the wavelength region WL3 and the information on the wavelength region WL4 to be individually acquired. As mentioned above, since different types of information related to the biological tissue for each wavelength region are included in the autofluorescence spectrum from the biological tissue, it is considered that the fact that information on different wavelength regions can be individually acquired is useful for the diagnosis.

As seen from the above, according to the embodiment, in the etalon-type optical unit capable of adjusting the wavelength when the transmittance reaches the peak, the full-width at half maximum of the peak P4a in the fourth wavelength region WL4 is increased. Therefore, it is possible to increase the amount of transmitted light of fluorescence, and to acquire a larger amount of information on the living body than ever before.
In addition, since the transmittance of the light blocking region around the peak P4a is low, it is possible to cut off light of this region, and to reduce noise.
When this etalon-type spectroscopic element 16 is embedded in an endoscope, it is possible to increase the difference between the fluorescence intensities depending on the existence of inflammation, a tumor, or the like. Thereby, it is possible to more easily discover inflammation or a tumor, or the like, and improve diagnostic accuracy.

### Example 1

Hereinafter, examples of the embodiment will be described.

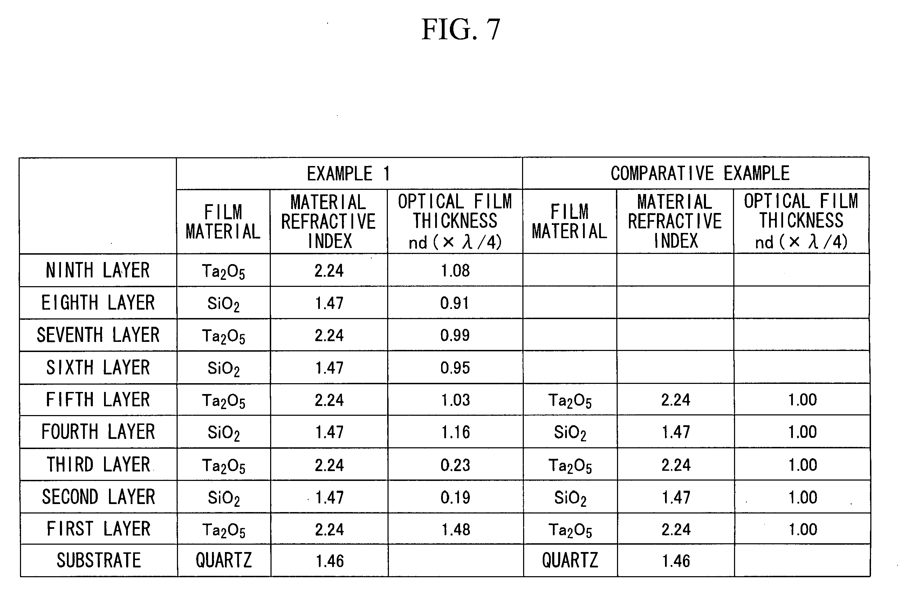

FIG. 7 shows structures of the optical elements 31 and 32 of the etalon-type spectroscopic element 16 according to example 1. In addition, a structure of a conventional optical element is shown in the drawing as a comparative example. With respect to any of the optical elements 31 and 32, a high refractive index film made of Ta₂O₅ as the first layer is formed on the quartz substrate 33 having a refractive index of 1.46. The refractive index of the first layer is 2.24, and the optical film thickness nd is 1.48 × (λ/4). Further, a low refractive index film made of SiO₂ as the second layer is formed on the first layer. The refractive index of the second layer is 1.47, and the optical film thickness nd is 0.19 × (λ/4). In addition, a layer made of Ta₂O₅ as the third layer is formed on the second layer, and the optical film thickness nd thereof is 0.23 × (λ/4). A layer made of SiO₂ as the fourth layer is formed on the third layer, and the optical film thickness nd thereof is 1.16 × (λ/4). A layer made of Ta₂O₅ as the fifth layer is formed, and the optical film thickness nd thereof is 1.03 × (λ/4). A layer made of SiO₂ as the sixth layer is formed on the fifth layer, and the optical film thickness nd thereof is 0.95 × (λ/4). A layer made of Ta₂O₅ as the seventh layer is formed on the sixth layer, and the optical film thickness nd thereof is 0.99 × (λ/4). A layer made of SiO₂ as the eighth layer is formed on the seventh layer, and the optical film thickness nd thereof is 0.91 × (λ/4). A layer made of Ta₂O₅ as the ninth layer is formed on the eighth layer, and the optical film thickness nd thereof is 1.08 × (λ/4).
Meanwhile, the design wavelength λ of the example is 700 nm, and units of the above-mentioned optical film thickness nd are all nm (nanometer).

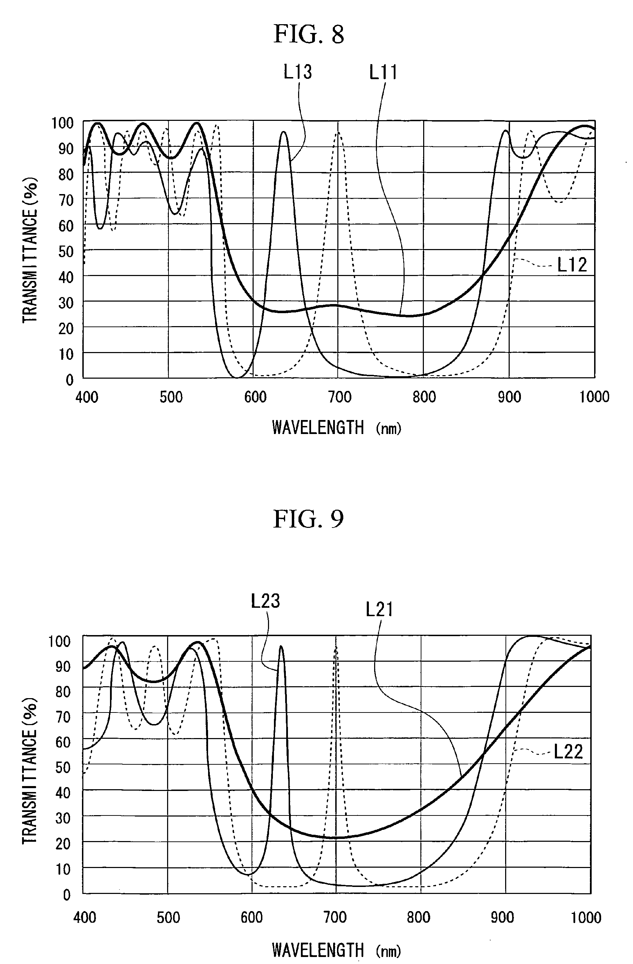

FIG. 8 shows the transmittance characteristics of the optical elements 31 and 32 manufactured in this way, and the transmittance characteristic of the etalon-type spectroscopic element 16 in which two optical elements 31 and 32 are disposed opposite to each other. The horizontal axis of the graph in FIG. 8 indicates a wavelength (nm), and the vertical axis thereof indicates a transmittance (%). The transmittance characteristic of only any one of the optical elements 31 and 32 is shown by the thick solid line L1 in FIG. 8. As shown in FIG. 8, the optical element 31 or the optical element 32 has a transmittance characteristic with a transmittance of 50% or more in the wavelength region of less than about 570 nm, and the wavelength region of about 900 nm or more. Further, the optical element has a transmittance characteristic with a transmittance of 30% or so in the wavelength region of about 570 nm to about 900 nm. In this wavelength region, one gentle and small peak appears, and as a result two valleys appear, one before the peak and one after the peak. The lowest transmittance of the valley portions is 25.8%, the peak transmittance is 28.4%, and the difference thereof is 2.6%.

Meanwhile, a method of designing two optical elements 31 and 32 which are disposed opposite to each other do not limited to an example shown in FIG. 7, and may be designed to generate the above-mentioned peak by variously changing the number of laminated multilayer films in at least one of optical elements, the refractive index and film thickness of each film. In the case of the etalon-type optical element, two optical elements disposed opposite to each other are all formed to have the same structure, and they are preferably disposed in a plane-symmetric manner. Thereby, the transmittance in the peak increases significantly.

The transmittance characteristic of the etalon-type spectroscopic element 16, in which the optical elements 31 and 32 are disposed opposite to each other, in the case of the state A1 (thickness of 520 nm of the air layer LG) is shown by the thin solid line L13 in FIG. 8, and the transmittance characteristic thereof in the case of the state A2 (thickness of 720 nm of the air layer LG) is shown by the dashed line L12 in FIG. 8. As shown in FIG. 8, in the case of the state A1, the etalon-type spectroscopic element 16 has a high transmittance in the wavelength region of less than about 570 nm, and shows a large peak around 630 nm. The full-width at half-maximum in this peak is 33 nm. In addition, the lowest transmittance of the light blocking region appearing around the peak is extremely low to be 1.0% or less.

On the other hand, in the conventional optical element, the high refractive index film made of Ta₂O₅ as the first layer is formed on the quartz substrate having a refractive index of 1.46. The refractive index of the first layer is 2.24, and the optical film thickness nd thereof is 1.00 × (λ/4). Further, the low refractive index film made of SiO₂ as the second layer is formed on the first layer. The refractive index of the second layer is 1.47, and the optical film thickness nd thereof is 1.00 × (λ/4). In addition, a layer made of Ta₂O₅ as the third layer is formed on the second layer, and the optical film thickness nd thereof is 1.00 × (λ/4). A layer made of SiO₂ as the fourth layer is formed on the third layer, and the optical film thickness nd thereof is 1.00 × (λ/4). A layer made of Ta₂O₅ as the fifth layer is formed on the fourth layer, and the optical film thickness nd thereof is 1.00 × (λ/4). The design wavelength λ in this case is 700 nm.
FIG. 9 shows the transmittance characteristics of the conventional single optical elements manufactured in this way, and the transmittance characteristic of the etalon-type spectroscopic element in which these conventional optical elements are disposed opposite to each other in pairs. The horizontal axis of the graph in FIG. 9 shows a wavelength (nm), and the vertical axis thereof shows a transmittance (%). The transmittance characteristic of the above-mentioned conventional single optical element is shown by the thick solid line L21 in FIG. 9. As shown in FIG. 9, the conventional optical element has a transmittance characteristic with a transmittance of 20% to 30% in the wavelength region of about 570 nm to about 900 nm, similarly to example 1. However, unlike example 1, one valley appears over the entire wavelength region of about 570 nm to about 900 nm, and the peak does not appear.

The transmittance characteristic of the etalon-type spectroscopic element, in which two conventional optical elements are disposed opposite to each other, in the case of the state A1 (thickness of 510 nm of the air layer LG) is shown by the thin solid line L23 in FIG. 9, and the transmittance characteristic thereof in the case of the state A2 (thickness of 700 nm of the air layer LG) is shown by the dashed line L22 in FIG. 9. As shown in FIG. 9, in the case of the state A1, the etalon-type spectroscopic element formed of the conventional optical elements shows a peak at around 630 nm, and has a full-width at half-maximum of 15 nm in this peak. In addition, the lowest transmittance of the light blocking region appearing before and after the peak is 2.7%.

As seen from the above, according to the etalon-type spectroscopic element 16 of the example 1, since the full-width at half-maximum in the peak appearing around 630 nm is more than twice that of the comparative example, it is possible to increase the amount of transmitted light in the fourth wavelength region. In addition, since the lowest transmittance of the light blocking region appearing before and after the peak is lower than that of the comparative example, it is possible to exclude light other than a desired wavelength region further than ever before, and to reduce the measurement error attributed to the noise.

### Example 2

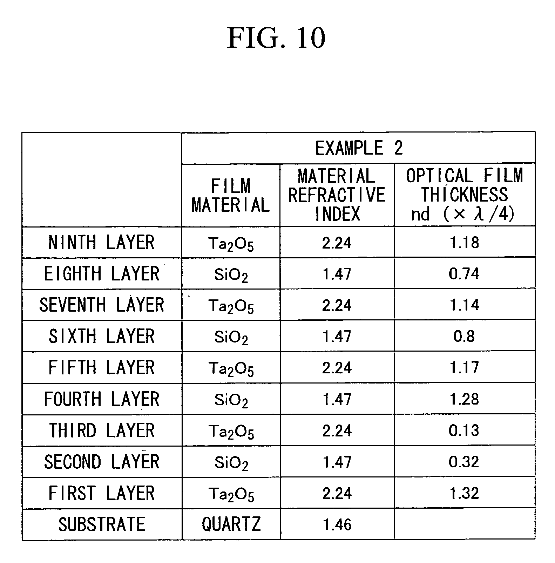

FIG. 10 shows structures of the optical elements 31 and 32 of the etalon-type spectroscopic element 16 according to example 2. With respect to any of the optical elements 31 and 32, a high refractive index film made of Ta₂O₅ as the first layer is formed on the quartz substrate 33 having a refractive index of 1.46. The optical film thickness nd of the first layer is 1.32 × (λ/4). Further, a low refractive index film made of SiO₂ as the second layer is formed on the first layer. The optical film thickness nd of the second layer is 0.32 × (λ/4). The refractive index of Ta₂O₅ is 2.24, and the refractive index of SiO₂ is 1.47. The refractive indexes are identical with respect to each of the layers below.
In addition, a layer made of Ta₂O₅ as the third layer is formed on the second layer, and the optical film thickness nd thereof is 0.13 × (λ/4). A layer made of SiO₂ as the fourth layer is formed on the third layer, and the optical film thickness nd thereof is 1.28 × (λ/4). A layer made of Ta₂O₅ as the fifth layer is formed on the fourth layer, and the optical film thickness nd thereof is 1.17 × (λ/4). A layer made of SiO₂ as the sixth layer is formed on the fifth layer, and the optical film thickness nd thereof is 0.8 × (λ/4). A layer made of Ta₂O₅ as the seventh layer is formed on the sixth layer, and the optical film thickness nd thereof is 1.14 × (λ/4). A layer made of SiO₂ as the eighth layer is formed on the seventh layer, and the optical film thickness nd thereof is 0.74 × (λ/4). A layer made of Ta₂O₅ as the ninth layer is formed on the eighth layer, and the optical film thickness nd thereof is 1.18 × (λ/4).
Meanwhile, the design wavelength X of the example is 700 nm, and units of the above-mentioned optical film thickness nd are all nm (nanometer).

FIG. 11 shows the transmittance characteristics of the optical elements 31 and 32 manufactured in this way, and the transmittance characteristic of the etalon-type spectroscopic element 16 in which two optical elements 31 and 32 are disposed opposite to each other. The horizontal axis of the graph in FIG. 11 shows a wavelength (nm), and the vertical axis thereof shows a transmittance (%). The transmittance characteristic of only any one of the optical elements 31 and 32 is shown by the thick solid line L31 in FIG. 11. As shown in FIG. 11, the optical element 31 or the optical element 32 has a transmittance close to 100% in the wavelength region of less than about 570 nm and the wavelength region of about 900 nm or more, and has a very high transmittance characteristic. Further, the optical element 31 or 32 has a transmittance characteristic with a transmittance of 30% or so in the wavelength region of about 570 nm to about 900 nm. In this wavelength region, one gentle and small peak appears, and as a result two valleys appear before and after the peak. The lowest transmittance of the valley portions is 28.6%, the peak transmittance is 38.0%, and the difference thereof is 9.4%.

The transmittance characteristic of the etalon-type spectroscopic element 16, in which the optical elements 31 and 32 are disposed opposite to each other, in the case of the state A1 (a thickness of 520 nm of the air layer LG) is shown by the thin solid line L33 in FIG. 11, and the transmittance characteristic thereof in the case of the state A2 (thickness of 720 nm of the air layer LG) is shown by the dashed line L32 in FIG. 11. As shown in FIG. 11, in the case of the state A1, the etalon-type spectroscopic element 16 has a high transmittance in the wavelength region of less than about 570 nm, and shows a large peak at around 630 nm. The full-width at half-maximum in this peak is 50 nm. In addition, the lowest transmittance of the light blocking region appearing before and after the peak is extremely low to be 1.0% or less.

As seen from the above, according to the etalon-type spectroscopic element 16 of example 2, since the full-width at half-maximum in the peak appearing around 630 nm is more than three times the comparative example shown in FIG. 9, it is possible to increase the amount of transmitted light in the fourth wavelength region. In addition, since the lowest transmittance of the light blocking region appearing before and after the peak is lower than that of the comparative example, it is possible to exclude light other than a desired wavelength region further than ever before, and to reduce the measurement error attributed to the noise. Further, the etalon-type spectroscopic element 16 of example 2 has a full-width at half-maximum larger than that of example 1. When comparing example 1 with example 2, the transmittance characteristics of the optical elements 31 and 32 show that as the transmittance of the peak P1 in the second wavelength region WL2 is higher, the full-width at half-maximum in the same peak can be made larger.

As mentioned above, although the preferred embodiment of the invention has been described, the invention is not limited to the above-mentioned embodiment. Additions, omissions, substitutions and other modifications of the configuration can be made without departing from the gist or scope of the present invention.

In the invention, the above-mentioned multilayer film has a transmittance characteristic where a first peak with a maximum transmittance exists in the above-mentioned second wavelength region. Only one first peak may exist in the above-mentioned second wavelength region, and a plurality of first peaks may exist therein. As in the above-mentioned embodiment, it is often the case that the minimum values exist before and after the peak. Consequently, it is preferable that the optical element is designed so that more than one light (for example, fluorescence) having different maximum absorption wavelengths is included between each of the minimum values including the peak. Particularly, in the spectroscopic element for switching multi-wavelength light (for example, etalon-type optical element), an excellent optical unit is provided in which more than one light (for example, fluorescence) having a small difference between the maximum absorption wavelengths is repeatedly accurately separated. The optical device including the optical unit capable of quickly dispersing more than one light is suitable for an image inspection apparatus for inspecting a physical body containing substances, such as a biological tissue, of which the amount or activity can be changed at any time. The image inspection apparatus includes various types of endoscopes and microscopes. When the optical element of the invention is applied to small-sized spectroscopic element s such as an etalon-type optical element, it is possible to minimally suppress damage of a body to be inspected, and to obtain a high-quality multicolor image.

A light source unit which oscillates coherent light as excitation light may be used as a fluorescence endoscope device. In addition, the etalon-type spectroscopic element can also be used in other spectroscopic analyzers. Further, the etalon-type spectroscopic element is not limited to autofluorescence, and may be used in dispersion of other fluorescence or of normal light.

In the optical device of the above-mentioned embodiment, the transmittance characteristic of the spectroscopic element composed of the optical element 31 and the optical element 32 is changed by changing the gap between the optical element 31 and the optical element 32 from L1 to L2. Incidentally, it is possible to increase the range of choice for the transmittance characteristic of the spectroscopic element by changing the gap between the optical element 31 and the optical element 32 to more than two multiple stages.

### Industrial Applicability

The present invention relates to an optical element including a multilayer film which has a first transmittance characteristic in a first wavelength region, and has a second transmittance characteristic in a second wavelength region existing at the side where a wavelength is longer than that of the first wavelength region, wherein the multilayer film has a transmittance characteristic where a first peak with a maximum transmittance exists in the second wavelength region.
The optical element of the present invention has a large full-width at half-maximum in the peak, and thus can be suitably used in a spectroscopic analysis.

## Claims

1. An optical element comprising:
a multilayer film which has a first transmittance characteristic in a first wavelength region, and has a second transmittance characteristic in a second wavelength region existing at the side where a wavelength is longer than that of the first wavelength region,
wherein the multilayer film has a transmittance characteristic where a first peak with a maximum transmittance exists in the second wavelength region.

2. The optical element according to Claim 1, wherein the transmittance in the first peak is higher than the lowest transmittance in the second wavelength region, and the difference thereof is 2% or more.

3. The optical element according to Claim 1, wherein when two optical elements are disposed opposite to each other, these two optical elements have a third transmittance characteristic in a third wavelength region, and have a fourth transmittance characteristic in a fourth wavelength region existing at the side where a wavelength is longer than that of the third wavelength region, and
the first peak of the optical element exists in the fourth wavelength region.

4. A spectroscopic element comprising the two optical elements according to Claim 1,
wherein two optical elements are disposed opposite to each other,
the spectroscopic element has a third transmittance characteristic in a third wavelength region, and has a fourth transmittance characteristic in a fourth wavelength region existing at the side where a wavelength is longer than that of the third wavelength region, and
the first peak of the optical element exists in the fourth wavelength region of the spectroscopic element.

5. An optical unit comprising:
the two optical elements disposed opposite to each other according to Claim 3; and
an adjustment mechanism that adjusts a gap along a light path of the optical elements,
wherein the adjustment mechanism performs switching of a wavelength in a wavelength region which has a higher transmittance than the lowest transmittance in the second wavelength region and includes the first peak.

6. The optical unit according to Claim 5, wherein the optical unit has the third transmittance characteristic in the third wavelength region, and has a fourth transmittance characteristic where a second peak with a maximum transmittance exists at the fourth wavelength region of the longer wavelength side than that of the third wavelength region, and
a full-width at half-maximum in the second peak is 30 nm or more, and the lowest transmittance in the fourth wavelength region is 1% or less.

7. An optical device comprising:
the optical unit according to Claim 5;
a light source unit that irradiates an object with illumination light;
an optical system that forms an image of the object; and
an imaging unit that captures the image,
wherein the optical unit is disposed between the optical system and the imaging unit.

8. The optical device according to Claim 7, wherein the optical unit is an etalon-type spectroscopic element.

9. The optical device according to Claim 8, wherein an optical element of the optical unit transmits fluorescence emanated from an object, and
the light source unit irradiates the object with excitation light for exciting the fluorescence.
